# EUROPEAN PATENT SPECIFICATION

(11) **EP 3 297 550 B1**
(45) Date of publication and mention of the grant of the patent: **28.11.2018**
(21) Application number: 16735683.1
(22) Date of filing: 19.05.2016
(51) Int. Cl.: A61B 17/42, A61F 2/00

(54) **SURGICAL DEVICE FOR IMPLANTING A MESH**
CHIRURGISCHE VORRICHTUNG ZUR IMPLANTATION EINES NETZES
DISPOSITIF CHIRURGICAL POUR IMPLANTATION D'UN TREILLIS

(30) Priority: 21.05.2015 IT UB20150783
(43) Date of publication of application: 28.03.2018
(73) Proprietor: Longo, Antonio, 90134 Palermo (PA) (IT)
(72) Inventor: Longo, Antonio, 90134 Palermo (PA) (IT)
(74) Representative: Papa, Elisabetta
(86) International application number: PCT/IB2016/052946
(87) International publication number: WO 2016/185422

(56) References cited:
- EP-A1- 0 543 499
- WO-A2-01/26724
- WO-A2-2009/104182
- US-A1- 2011 054 249
- US-A1- 2013 012 966

## Description

### Technical field of the invention

The present invention relates to a surgical device provided for treating the prolapse of one or more pelvic organs, for example: uterus, vagina, bladder, rectum.

### Background

The prolapse of the pelvic organs, in particular of uterus and bladder, represents a sanitary problem with growing proportions. Currently, the prolapse of uterus and bladder is treated by urogynecologists by trans-vaginal or trans-abdominal route. Almost all these operations provide hysterectomy and the positioning of *mesh,* or net, between bladder and vagina and vagina and rectum. The *meshes* positioned in such seats cause frequent, and sometimes, serious complications, so that the US FDA (*Food* & *Drug Administration*) has drawn the attention to this problem. Furthermore, such operations are burdened with a very high percentage of recidiva.
The colorectal surgeons treat the inner or outer prolapse of rectum by transanal or trans-abdominal route. Tons of types of such operations, above all by abdominal route, have been proposed. This demonstrates the unsatisfactory results. In particular, the proctopexy operations are burdened with a high percentage of serious constipation and other surgical complications; the transanal operations are criticized as they can cause faecal incontinence and frequent recidiva.

For 10 years the inventor has been proposing a wholly new and less invasive technique than traditional operations. The technique is known with the POPS (*Pelvic Organs Prolapse Suspension*) acronym and the main peculiarities are: uterus is not removed if it is healthy; *meshes* are not interposed between the pelvic organs; the prolapse of all prolapsed pelvic organs is corrected at the same time; it can be performed in laparoscopy. A variant called SIR (*Soft Intraperitoneal Recto-suspension*) is suitable when the prolapse involves rectum only.
The POPS technique consists in suspending the prolapsed pelvic organs at the side walls of the abdomen, by means of a tape-like *mesh* cut like a V, the corner or centre is fastened to the pelvic organs, the branches' ends to the bands of the abdomen's side walls. The *mesh* is positioned in the subperitoneum through a suitably implemented tunnel.
The technique carrying-out has some critical aspects. In particular, the creation of the subperitoneal tunnel and the crossing of the wide ligaments of the uterus is not easy. Currently, it is performed with conventional instruments adapted as tunnelling devices, which however need assisting manoeuvres of other laparoscopic instruments. Furthermore, there is a discrete risk of damaging the subperitoneal structures, as it is not possible performing a decortication of the peritoneum before crossing it with the tip of the used pliers. Still, suturing the *mesh* to the pelvic organs is not an easy surgical manoeuvre. Such difficulties cause some risks, prolong the operating time and make the technique not well standardisable.

A device for deploying a mesh and expanding tissue is disclosed in WO01/26724 A2.

### Summary of the invention

The technical problem placed and solved by the present invention is then to provide a surgical device allowing to obviate the drawbacks mentioned above with reference to the known art, and in particular to perform the above-mentioned surgical procedures POPS and SIR.
Such problem is solved by a device according to claim 1.
Preferred features of the present invention are subject of the depending claims.
The invention provides a device, or instrument, configured for being inserted in the abdomen through a cutaneous incision and for forwarding below the peritoneum and between the wide ligaments, as far as the vaginal vault. The device allows positioning a *mesh* in the path comprised between the abdominal incision and the pelvic organs involved by the prolapse. The *mesh* is fastened to the vagina with one or more points, or staples, the latter preferably applied by means of the device itself. At the end of the operation, advantageously the *mesh* can be positioned in the sub-peritoneal tunnel as far as the cutaneous incision, wherein it is fastened to the muscle bands.
Therefore, the device is suitable: to create a subperitoneal tunnel comprised between the abdominal wall and the prolapsed pelvic organs; to position a band-like suspension *mesh*, or net, in the created subperitoneal tunnel; to fasten an end of the *mesh* to the prolapsed organs and the other end to the muscle bands of the abdomen.
The device also comprises a distal tunnelling device, or canalizing element or dissector, which, by extending and contracting, eases the separation of the peritoneum from the abdominal bands and the separation of the two peritoneal layers of the wide ligaments, as far as reaching the vaginal vault. Such dissecting means, which can be defined even simply as dissector, canalizing element or tunnelling device, preferably is activated by means of an actuation placed at a proximal hand grip of the device.
Preferably, the canalizing means comprises one or more selectively deformable elements, in particular in direction orthogonal to the forward longitudinal one of the device inside the abdomen.

Based upon a particularly preferred embodiment, the device further comprises a suction system. The latter is capable of performing a distal suction drawing, that is sucking, the tissue of the vaginal vault inside a distal opening, placed at the tip of the device. Such suction action allows the adhesion of the *mesh* included in the device to the sucked vaginal vault and then allows applying points or staples on such elements, by fastening one to the other one.

Preferably, the device has an elongated main body with anatomical shape, reproducing the profile of the abdominal body district which is crossed to access the vaginal vault. In particular, preferably the main body develops longitudinally with a distal angulation imitating the anatomic curve comprised between the lateral wall of the abdomen and the vaginal vault.

In preferred embodiments, the device comprises a distal end shaped like a tip, preferably with triangular section, which eases the insertion thereof through the cutaneous incision and it eases the separation of the peritoneum, and in general of the tissues crossed during the forwarding inside the abdomen.

The invention then provides an instrument dedicated to the surgical operation for treating the prolapse of the pelvic organs. Such device makes the POPS repeatable, standardisable, faster, reliable and less invasive. The risk of damaging the tissues and the subperitoneal structures is reduced. It makes the operation performable, wherein additional manoeuvres or operations are not required, in video-laparoscopy with only optical assistance, thus by avoiding the abdominal incisions for inserting other laparoscopic instruments. Then, it allows a considerable saving in consumption materials.

Other advantages, features and use modes of the present invention will result evident from the following detailed description of some embodiments, shown by way of example and not for limitative purpose.

### Brief description of the figures

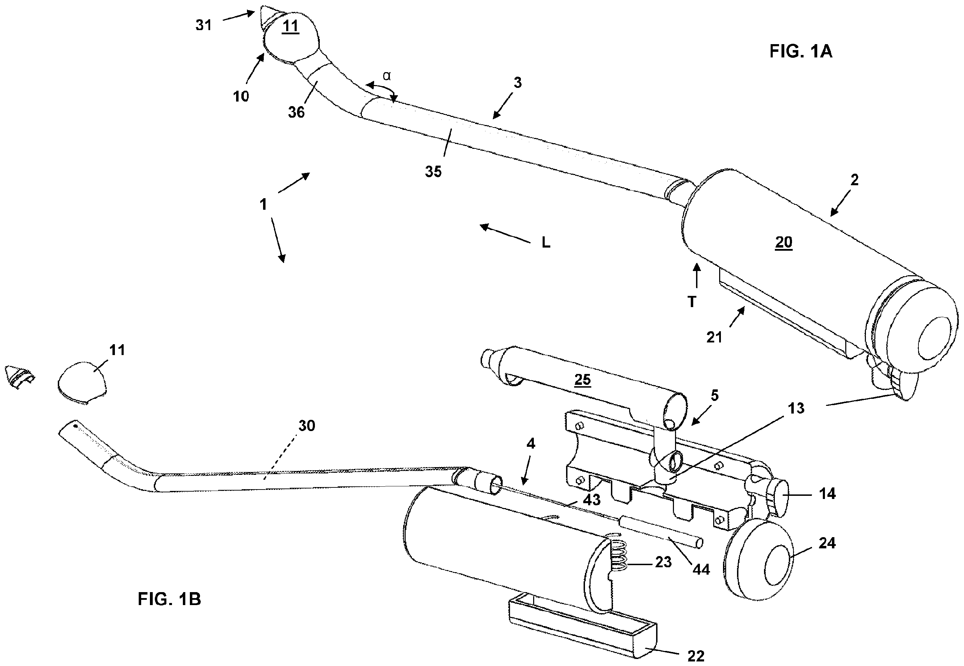

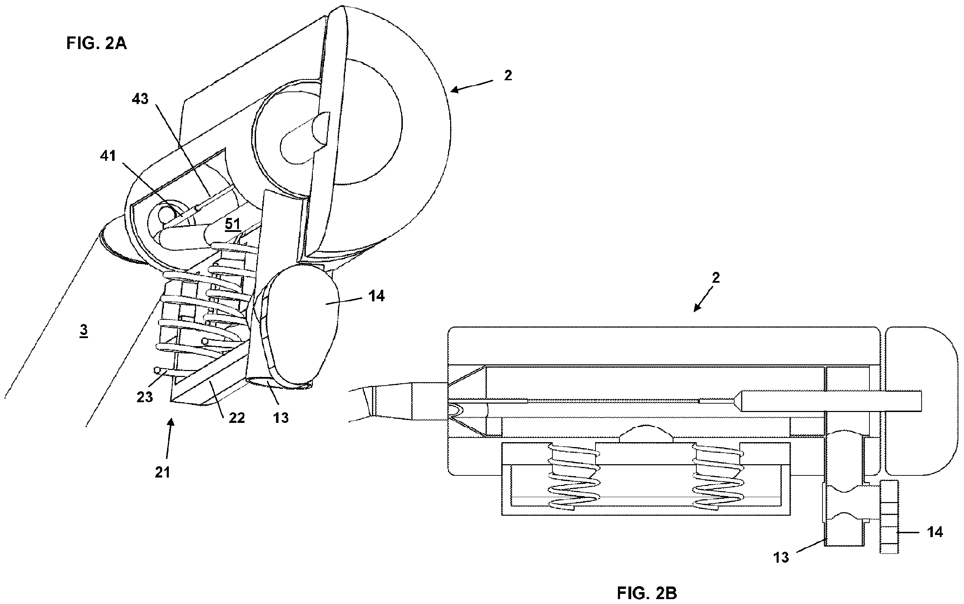

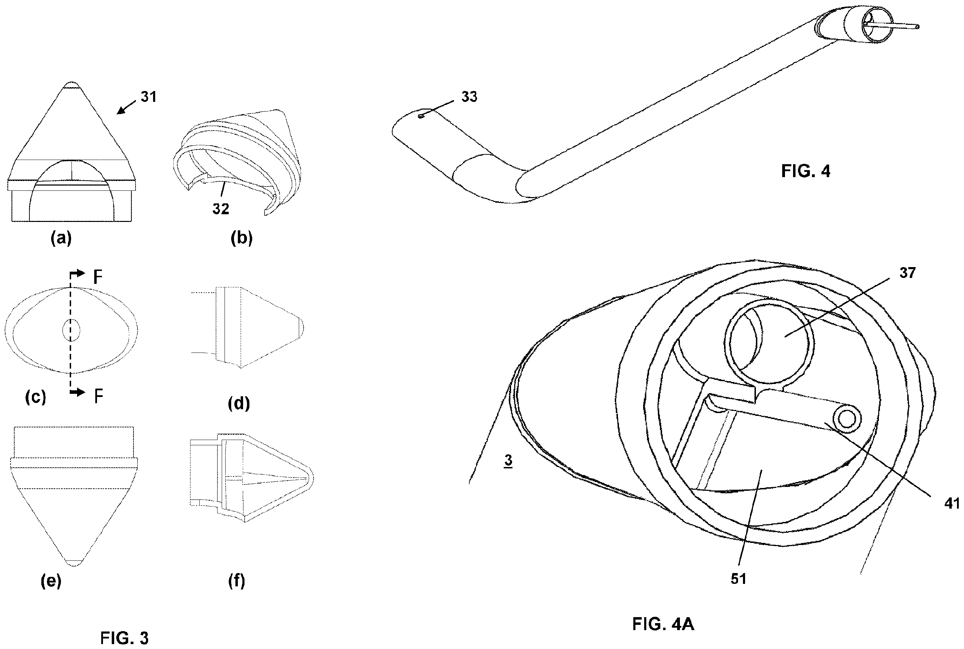

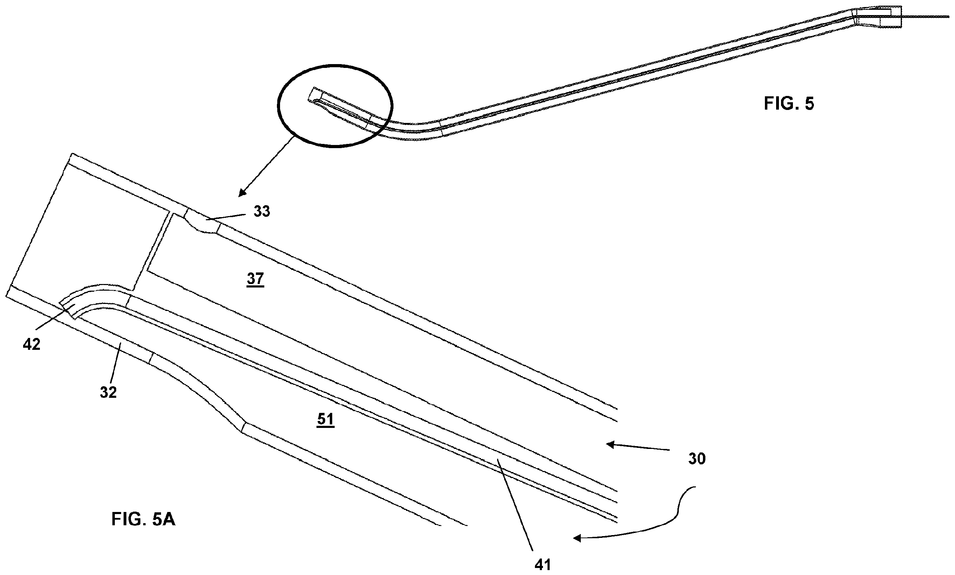

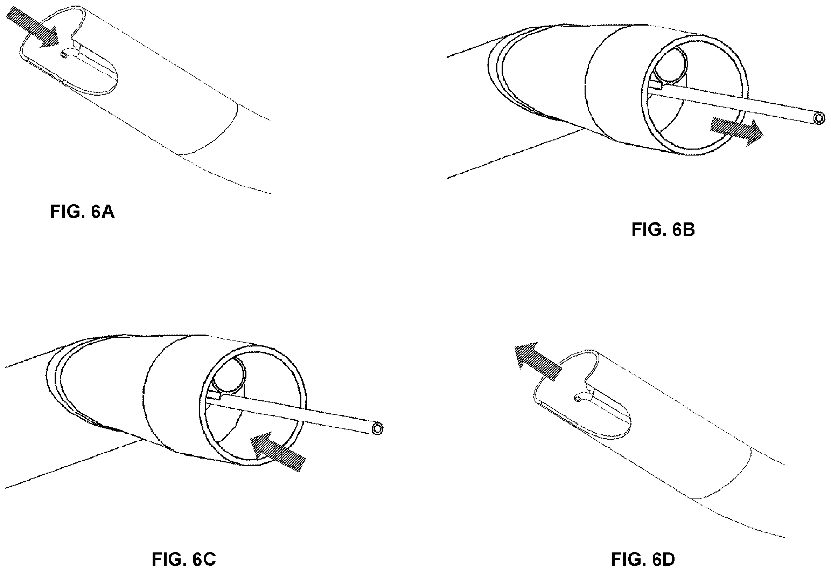

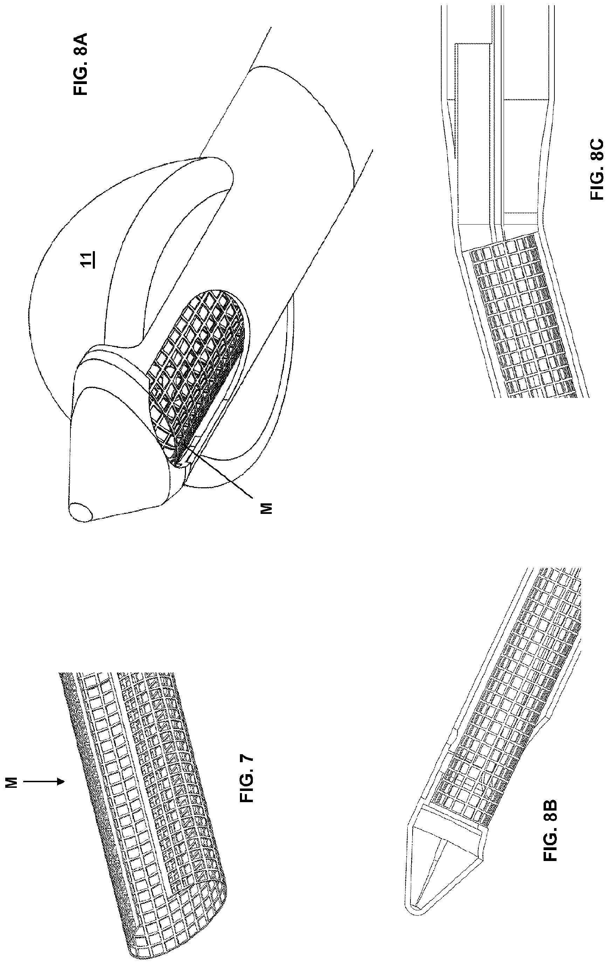

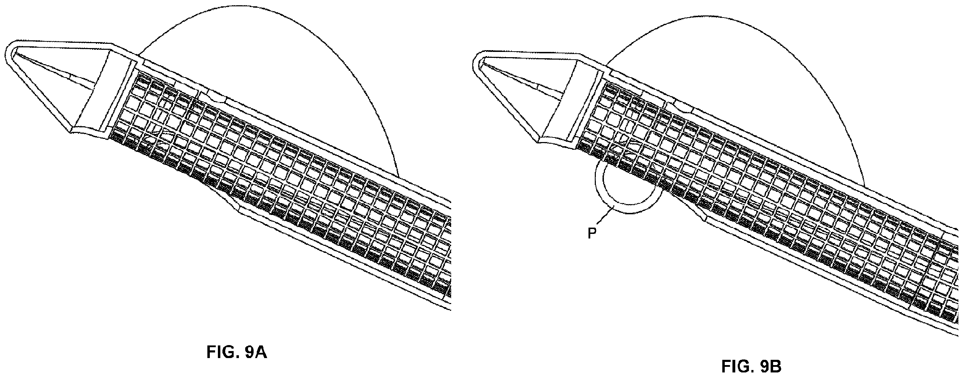

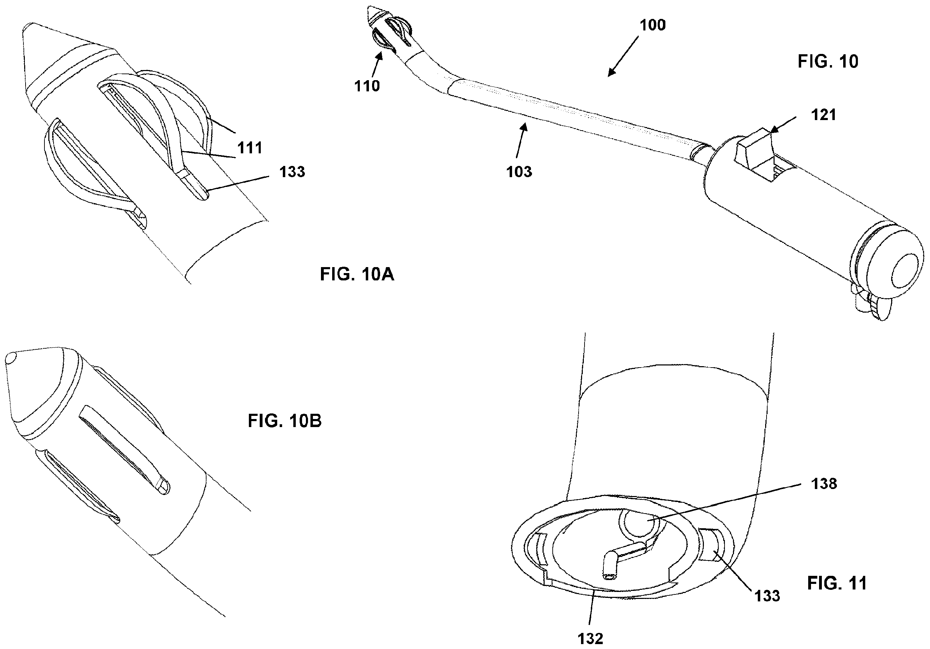

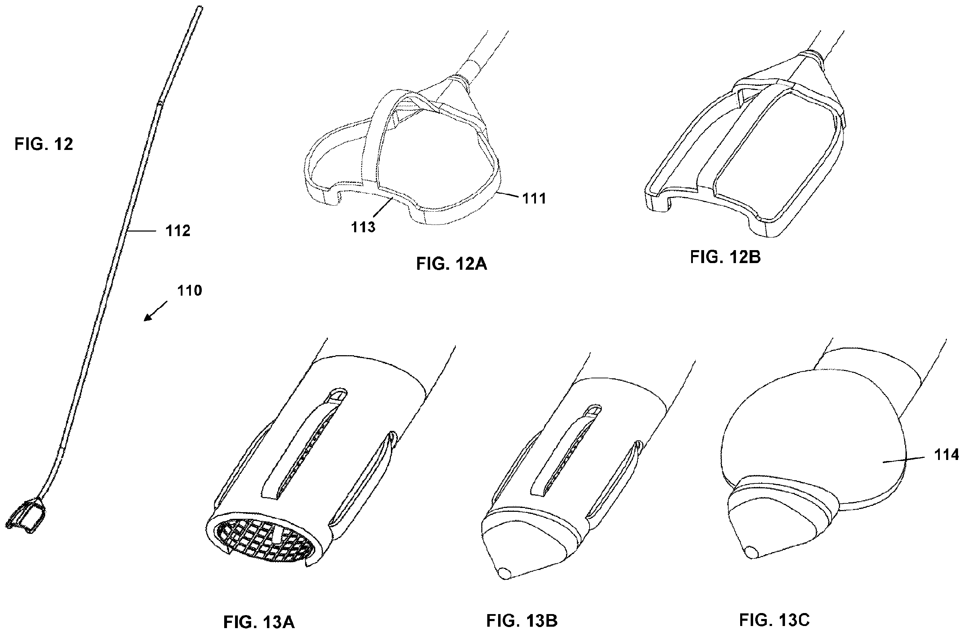

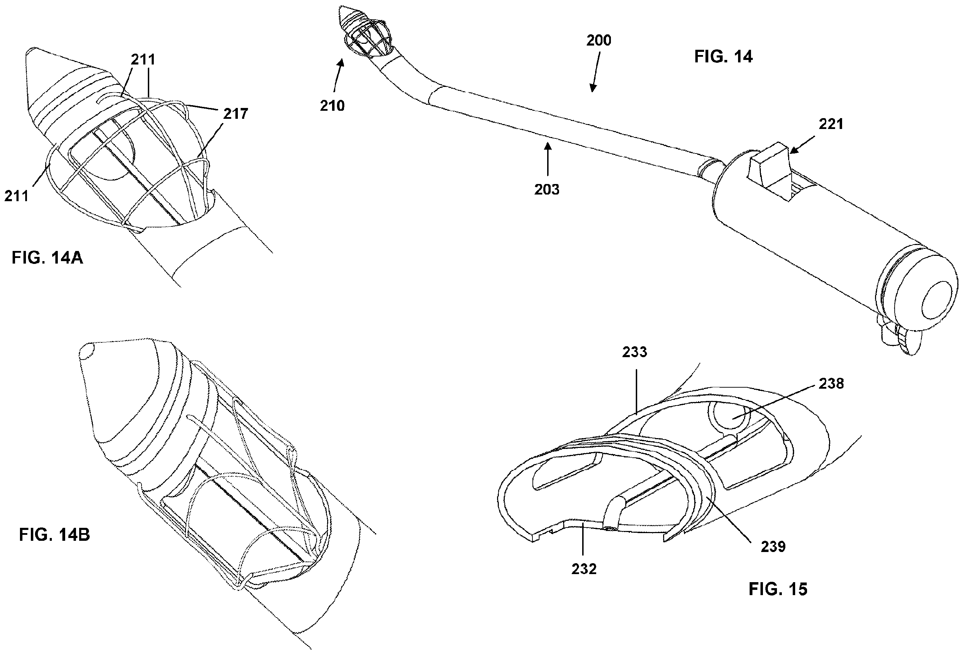

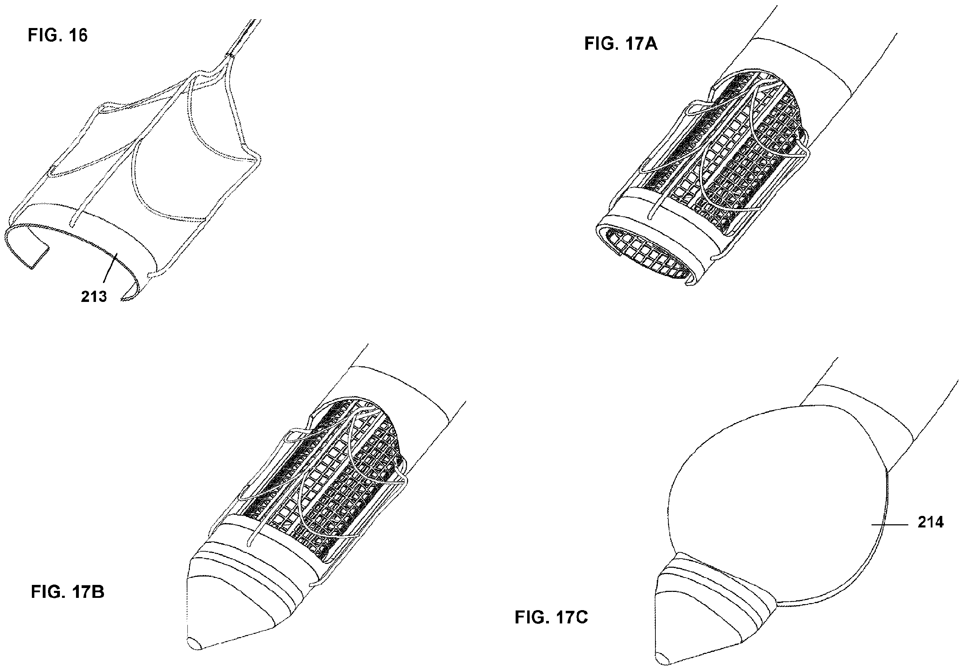

The figures of the enclosed drawings will be referred to, wherein:
▪ Figures 1A and 1B relate to a surgical device for applying a supporting element on the vaginal tissue based upon a first preferred embodiment of the present invention, by showing a perspective view and an exploded view thereof, respectively;
▪ Figures 2A and 2B relate to a proximal portion of the device of Figure 1, by showing a partially cut-out rear perspective view and a side view thereof, with removal of some components for making visible inside, respectively;
▪ Figure 3 shows a distal end, or tip, of the device of Figure 1 in different views, in particular: (a) in a first side view, (b) in perspective, (c) in top plan, (d) in a second side view, (e) in a third side view, (f) in a view in section performed according to the line F-F of the view (c);
▪ Figure 4 shows a perspective view of a portion of a main body of the device of Figure 1 and Figure 4A shows an enlarged detail of the view of Figure 4;
▪ Figure 5 shows a view in longitudinal section of a portion of a main body of the device of Figure 1 and Figure 5A shows an enlarged detail of the view of Figure 5;
▪ Figures 6A to 6D relate to a mode for inserting the supporting element into the device of Figure 1, by showing, each one, a respective perspective view of a proximal or distal end portion of the main body of such device;
▪ Figure 7 shows a supporting element, or *mesh,* which can be inserted into the device of Figure 1;
▪ Figures 8A to 8C related to the device of Figure 1 in a configuration wherein the supporting element of Figure 7 is inserted inside the device itself, by showing a perspective side view of a distal portion of the device, a view in longitudinal section of such distal portion and a view in longitudinal section of a proximal portion of the device, respectively;
▪ Figures 9A and 9B relate, each one, to a respective step of applying *in situ* the supporting element, by showing each one a view in longitudinal section of a distal portion of the device of Figure 1;
▪ Figures 10A to 10C relate to a surgical device for applying a supporting element on the vaginal tissue based upon a second preferred embodiment of the present invention, by showing a perspective view of the device, an enlarged view of a distal portion thereof in a first configuration and an enlarged view of such distal portion in a second configuration, respectively;
▪ Figure 11 shows a perspective view of a distal portion of a main body of the device of Figure 10A;
▪ Figures 12 to 12B relate to a canalizing or dissecting element of the device of Figure 10A, by showing a perspective view of such element, an enlarged view of a distal portion thereof in a first configuration and an enlarged view of such distal portion in a second configuration, respectively;
▪ Figures 13A to 13C relate to the distal portion of the device of Figure 10A, by showing each one a perspective view thereof in a respective step for inserting a supporting element and assembling;
▪ Figures 14A to 14C relate to a surgical device for applying a supporting element on the vaginal tissue based upon a third preferred embodiment of the present invention, by showing a perspective view of the device, an enlarged view of a distal portion thereof in a first configuration and an enlarged view of such distal portion in a second configuration, respectively;
▪ Figure 15 shows a perspective view of a distal portion of a main body of the device of Figure 14A;
▪ Figure 16 relates to a canalizing or dissecting element of the device of Figure 14A, by showing a distal portion thereof in said second configuration; and
▪ Figures 17A to 17C relate to the distal portion of the device of Figure 14A, by showing each one a perspective view thereof in a respective step for inserting a supporting element and assembling.

The thicknesses and bends represented in the above-illustrated figures are to be meant by pure way of example, they are generally magnified and not necessarily shown in proportions.

### Detailed description of preferred embodiments

Several embodiments and variants of the invention will be described hereinafter and this with reference to the above-mentioned figures.
Analogous components are designated in the different figures with the same reference numeral.
In the following detailed description, additional embodiments and variants with respect to embodiments and variants already treated in the same description will be illustrated limited to the differences with respect to what already illustrated.
Furthermore, the different embodiments and variants described hereinafter are susceptible to be used in combination, where compatible.

By firstly referring to Figures 1A and 1B, a surgical device or instrument according to a first embodiment of the invention is designated as a whole with 1.
The device 1 è configured for inserting a supporting element, in particular a *mesh* or net, at the vaginal vault to treat the prolapse thereof.

The device 1 mainly comprises:
▪ a proximal handpiece 2 which can be held/grasped by a user;
▪ an elongated main body 3, fixedly connected to the handpiece 2 and configured for being inserted percutaneously through the abdomen as far as the vaginal vault;
▪ canalizing means 10, arranged at a distal end portion of the main body 3 and based, in the present example, upon a balloon 11 projecting from the main body itself and which can be selectively inflated and deflated to ease the separation of abdominal tissues during the insertion of the main body 3 through the abdomen of the patient;
▪ means 4 for applying a staple or fixing point of the *mesh* to the vaginal tissue, which staple or point typically is metallic;
▪ a suction system 5, configured in such a way as to apply a sucking action on the vaginal vault at the distal portion of the main body 3.
Each one of the above-mentioned components will be now described in greater detail.

As it will be illustrated shortly, the proximal handpiece 2, in general terms, has elements for actuating the canalizing means 10, the means 4 for applying a staple and the suction system 5, which can be actuated by an operator with his/her hand.
Moreover, at the level of the handpiece 2 a connection 13 is provided, which can be connected to a suction system and/or pneumatic power supply existing in the operating environment. Typically, the connection 13 is equipped with a tap 14, occlusion valve or other element with analogous function.
The handpiece 2 has a main portion 20 which can be grasped by the user, for example with cylindrical and/or elongated geometry.

The main body, preferably, is hollow inside and it has at least a longitudinal room 30 apt to house the *mesh.* Under "longitudinal direction", in the present context, a prevailing development direction of the device 1, and in particular of the main body thereof 3, is meant. Such direction is designated with L in Figure 1A. Such direction also corresponds substantially to the direction for inserting and forwarding the main body 3 in the abdomen of the patient.
However, it is to be noted that, advantageously, the main body 3 does not extend according to one single rectilinear direction, but it has a corrugated development, by extending so as to reproduce an anatomic curve comprised between the lateral wall of the abdomen and the vaginal vault. In the present example at least two mutually tilted portions of the main body 3 can be seen, and in particular a first more proximal 35 and a second more distal portion 36 forming an obtuse angle a.
The main body 3 has a tip-like shaped distal end 31, represented in detail in Figure 3 and shaped so as to ease the insertion of the main body itself inside the abdomen of the patient and the separation/dissection of the tissues gradually met as far as the site of interest, that is the vaginal vault. In the present example, such portion 31 is shaped like a trilobate tip. Such tip 31 is placed in the position immediately distal to the above-mentioned balloon 11. The tip-like shaped portion 31 preferably is hollow and however it contributes to define an opening or side door 32 of the main body 3 the function thereof will be explained hereinafter.

The balloon 11 can be selectively inflated and deflated, alternatively, thanks to a joint to the above-mentioned connection 13.
To this purpose, by also referring to Figures 4 and 4A, inside the main body a pneumatic supply channel 32 is obtained, leading distally into a door or hole 33 for feeding the balloon 11. Preferably, such door or hole 33 is arranged at a side skirt of the main body 3.
By also referring to Figures 2A and 2B, the channel 32 is also fed with inflating air of the balloon 11 by means of actuating means 21 which can be actuated by the operator and arranged on the handpiece 2. In the present example, the actuating means 21 comprises an actuation element 22 shaped like a button, advantageously elongated in L direction for an easier handling. The button 22 can be actuated by means of a pressure applied in a direction T substantially orthogonal to the prevailing development direction L of the handpiece 2, of the main body 3 and, generally, of the device 1. In this way, the button 21 can be actuated with the fingers of the hand while this is holding the handpiece 2.
Contrast elastic means is associated to the button 22, in the present example two compression helical springs 23.
In the herein considered preferred configuration, a pressing action on the button 22 determines the inflating of the balloon 11. When the button is released by the operator, the springs 23 bring back the button itself in a rest configuration corresponding to the deflating configuration of the balloon 11.
In this way, the operator can actuate alternatively the balloon 11 from a maximum encumbrance configuration, that is inflated and radially projecting from the main body 3, to a minimal encumbrance configuration, that is deflated and substantially adhering to the main body itself. Such alternation eases the dissection/separation of the tissues and then the forwarding of the main body 3 through the abdomen.
The inflating air can be sent inside the channel 32 and then to the balloon 11 by associating sealing components to the actuating means 21 allowing to make them to act like a kind of small pump. For example, the means 21 can act together with an air chamber 25.
Embodiment variants can provide that the channel 32 is placed selectively in communication with the connection 13, in this case used for feeding air under pressure of the system.
As it will be illustrated also hereinafter by referring to other embodiments of the invention, the canalizing means can also provide elements different from the above-mentioned balloon 11, however apt to assume, alternatively, a minimal encumbrance configuration and a maximum encumbrance configuration.
Embodiment variants can provide deformable elements in the sense that they have arrangements and/or mechanical connections suitable to allow the assumption of the above-mentioned minimum and maximum encumbrance configurations.

By mainly referring to Figures 1B, 4A and 5, 5A, the means 4 for applying a staple comprises an operative, or guiding channel 41 extending within the main body 3 for carrying the staple as far as a distal exit door or nozzle 42 which opens at the opening 32 of the main body 3. Inside the channel 41 a thread or other point-pushing element 43 is mobile, the latter preferably equipped with a widened actuation proximal portion 44.
The staple can be inserted into the device 1 at a loader thereof, which by simplicity has not been represented and which is suitable to include a plurality of staples and to feed them in sequence, upon control, inside the channel 41. In a simpler variant, a plurality of points is loaded directly inside the channel 41.
The application of the staple to the tissue is controlled by a proximal actuation or driver 24. Preferably, it is a button arranged at the longitudinal end of the handpiece 2 and which can be actuated by the operator by means of a pressure substantially oriented in longitudinal direction L. In particular, the button 24 is configured for being activated by the palm of the hand.

The suction system 5 too, preferably, can be joined selectively to the same above-mentioned connection 13. The system includes an operative channel 51, for example visible in Figure 4A and extending longitudinally inside the main body 3 as far as the distal opening 32 thereof.
The suction activation can be controlled directly by actuating the proximal tap 14 or by means of other dedicated element.

Figure 7 shows an example of *mesh,* herein designated with M, in the configuration wherein it is arranged inside the room 30 of the main body 3. In the represented example, the *mesh* has a basic shape with polygonal basis, in particular with rectangular strip, and it is housed inside the room 30 with the longitudinal margins folded towards inside.
Figures 6A to 6D exemplify the modes thereby the *mesh* M is arranged inside the device 1. In particular, as schematised in Figure 6A, at first in the distal portion of the main body 3 a long (for example about 40 cm) and flexible pliers is inserted, so as to cover the whole inner room 30 of the main body 3. As schematised in Figure 6B, once reached the proximal end of the main body 3, the pliers grasps a pre-cut portion of *mesh.* The pliers is then driven by dragging the mesh inside the main body 3 and, once reached again the distal end, the *mesh* is released from the pliers so as to let it to be trapped inside the room 30.
Preferably, the *mesh* is inserted in the main body 3 of the device 1 already in the assembling step and the instrument is sold ready for use.
In a possible embodiment, the *mesh* is pre-cut in portions of about 2.5cmx30cm and folded on itself to minimize the width thereof. As it is deformable, the *mesh* substantially assumes the shape of the room 30 housing it.

Figures 8A to 8C and 9A to 9B represent indeed the device 1 with the *mesh* M housed inside thereof.
As to the operative modes of the device 1 - and by referring to the just mentioned figures - the trilobate tip 21 eases the insertion of the device 1 through the cutaneous incision and it allows the separation of the peritoneum during the forwarding of the instrument inside the abdomen. Such separation is eased even by the repeated inflation of the balloon 11 actuated by the button 22.
Once reached the vaginal vault the suction of the system 5 is actuated and the vaginal wall is sucked back inside the opening 32 so as to make it to adhere against the *mesh* M.
The point-pushing button 24 is then actuated with the palm of the hand and the thread 43 forces the point, for example made of titanium and designated with P in Figure 9B, through the output section 42. The latter, advantageously, has a shape with strict radius of curvature, so as to impress a plastic deformation at the point outgoing from the operative channel 41 and which is induced to assume a circular shape, by grasping both the vaginal wall and the *mesh.*
By loading several points in series, it is possible then to apply them by simply actuating the button 24 repeatedly, by making them to exit one at time, prior the temporary suction exclusion and the repositioning of the device in a different (more proximal) point of the vaginal vault.
The instrument is then extracted from the patient and, whereas an end of the *mesh* remains fixed to the vaginal wall, the other one, once having extracted the instrument, is fastened to the abdominal muscles thus by implementing a so-called procedure of "*Pelvic Organ Prolapse Suspension*" (POPS).

Figures 10 to 13C relate to a second preferred embodiment of the device of the invention, herein designated as a whole with 100.
In this case, the canalizing means, designated with 110, include a plurality of selectively reversibly deformable elements 111 for passing from the above-mentioned maximum encumbrance configuration to the minimum encumbrance one and viceversa. In the present example, such deformable elements 111 are three and each one preferably in form of bar, in particular flat and with substantially rectangular plan profile. Based upon a preferred embodiment, the bars 111 are made of plastic material.
Each bar 111 is connected, at its own first proximal end, to the distal end of an actuating shaft 112. The other (distal) end of each bar 111 is constrained on a contrast element 113. This latter abuts onto, and thus is locked to, a corresponding inner profile, not shown, of the main body 103. The shaft 112 is slidingly housed inside an inner room 138 of the main body 103. Preferably, it is flexible to follow the longitudinal profile of the main body 103.
The whole group of the shaft 112, of the elements 111 and of the contrast 113 can be implemented integral.

In the maximum encumbrance configuration shown, for example, in Figures 10, 10A, 12A and 13C, each bar 111 outgoes from a respective slot 133 of the main body 103. Preferably, the slots 133 are arranged laterally to an opening or distal door 132 of the main body 103 with function analogous to the already described one.
The deformation of the bars 111 is induced by pushing longitudinally the shaft 112 by means of a proximal actuation 121 of lever-like type. The action onto the lever 121 is applied in substantially longitudinal direction and transmitted to the shaft 112 by means of mechanical connections of type known on itself. Even to the lever 121 contrast elastic means can be associated, apt to bring it back to a rest configuration (back, in the present embodiment) corresponding to a minimal encumbrance configuration of the means 110.
The minimum encumbrance configuration of the elements 111 is shown in Figures 12B and 13B.
As shown in Figure 13C, advantageously onto the bars 111 a wrapping membrane 14, for example made of silicone, can be applied to prevent the entry of biological residues into the slots 133 and to apply the inserting action in a more effective way without damaging the surrounding tissues.

Figures 14 to 17C relate to a third preferred embodiment of the invention device, herein designated as a whole with 200.
The device 200 provides canalizing means 210 with structure similar to that of the above-described second embodiment, wherein however the bars are replaced by longitudinal filiform elements 211, preferably made of metal. The filiform elements 211 are deformable selectively and in a reversible way, in a way analogous to what already illustrated for the bars, in particular by means of a proximal lever-like actuation herein designated with 221.
The filiform elements 211 can be associated, for greater stability, to transversal filiform elements, or crossbeams 217. Furthermore, even such elements 211 and 217 can be wrapped in a membrane 214 of the already described type.

The filiform elements 211 are associated to an actuation shaft analogous to the already described one, sliding in an operative channel 238 of the main body 203. Furthermore, at its own distal end they are constrained to a gripping element 213 in form of stirrup or sleeve. The latter is suitable to engage a corresponding seat 239 of the main body 203, by remaining retained by interlocking inside thereof. The filiform elements, in case in the form of grid associated to the presence of crossbeams 217, outgo from a common opening 233, preferably obtained on the side skirt of the main body 203 on opposite side with respect to an opening 232 thereof with function analogous to the already described one.

Even if the device of the invention has been sofar described by referring to the treatment of the vaginal prolapse, other surgical applications cannot be excluded.

The present invention has been sofar described with reference to preferred embodiments. It is to be meant that other embodiments belonging to the same inventive core may exist, as defined by the protective scope of the herebelow reported claims.

## Claims

1. A surgical device (1; 100; 200), configured for inserting a supporting element (M), preferably a *mesh,* at the vaginal vault, at the rectum or at any other pelvic organ to treat the prolapse thereof, which device comprises:
- a proximal handpiece (2) apt to be held by an operator;
- an elongated main body (3), connected or fixedly connectable to said handpiece (2) and arranged distally with respect thereto, which main body (3) is suitable to be percutaneously inserted through the abdomen up to the vaginal vault or the other organ ;
- canalizing means (10), arranged at a distal end portion (31) of said main body (3) and operable at said proximal handpiece (2), which canalizing means (3) is apt to assume, alternatively, a minimal encumbrance configuration and a maximum encumbrance configuration apt to favour the separation of abdominal tissues during the insertion of said main body (3) through the abdomen, **characterized in that** the main body (3) has a longitudinal room (30) apt to accommodate the supporting element (M).

2. The device (1; 100; 200) according to claim 1, wherein said main body (3) has a waved shape, extending longitudinally according to a profile apt to reproduce an anatomic curve comprised between the lateral wall of the abdomen and the vaginal vault.

3. The device (1; 100; 200) according to anyone of the preceding claims, wherein said main body (3) has a distal end with a pointed shape (31), in particular trilobate, apt to favour the insertion of the device through the abdominal tissues.

4. The device (1) according to anyone of the preceding claims, wherein said configurations of maximum and minimum encumbrance of said canalizing means (10) correspond to a maximum extension and a minimum extension thereof, respectively, in a transverse direction orthogonal to a longitudinal direction of forward motion of said main body (3) through the abdomen.

5. The device (1) according to anyone of the preceding claims, wherein said canalizing means (10) comprises one or more elements (11) selectively deformable to assume said configurations of maximum and minimum encumbrance.

6. The device (1) according to anyone of the preceding claims, wherein said canalizing means (10) comprises a membrane or balloon (11) which is selectively inflatable.

7. The device (100; 200) according to anyone of claims 1 to 5, wherein said canalizing means (110) comprises a plurality of reversibly deformable elements (111), preferably configured to selectively exit each one from a respective opening (133) obtained in a side skirt of said main body (103) to assume said maximum encumbrance configuration.

8. The device (100; 200) according to the preceding claim, wherein said deformable elements comprise a plurality of longitudinal bar elements (111), preferably made of plastic material, or a plurality of longitudinal filiform elements (211), preferably made of metal.

9. The device (200) according to claim 7 or 8, wherein said deformable elements have a grid configuration formed by longitudinal elements (211) and transverse elements (217).

10. The device (100; 200) according to anyone of claims 7 to 9, comprising a shaft (112) for actuating said reversibly deformable elements (111), which actuating shaft (112) extends longitudinally inside said main body (103), wherein preferably said reversibly deformable elements (111) are arranged distally with respect to said actuating shaft (112) and secured thereto.

11. The device (1; 100; 200) according to anyone of the preceding claims, comprising means (21; 121) for actuating said canalizing means (10), which actuating means (21; 121) is arranged at said proximal handpiece (2), it can be activated by an operator and preferably it comprises a lever or button, which actuating means (21; 121) preferably is configured to be activated by a force applied in a direction transversal or longitudinal to the device.

12. The device (1; 100; 200) according to the preceding claim, wherein said actuating means (21) comprises an actuation element (22) and contrast elastic means (23) associated thereto, which contrast elastic means (23) is apt to bring back said actuating element (22) in a rest configuration corresponding to said minimal encumbrance configuration of said canalizing means (10).

13. The device (1; 100; 200) according to anyone of the preceding claims, comprising means (4) for applying a staple or fixing point of the supporting element to the vaginal vault or other organ.

14. The device (1; 100; 200) according to the preceding claim, wherein said means (4) for applying a staple comprises a guiding channel (41) for the staple extending within said main body (3) and preferably provided with an exit port (42) of the point arranged at a distal opening (32), preferably a side opening, of said main body (3).

15. The device (1; 100; 200) according to claim 13 or 14, wherein said means (4) for applying a staple comprises a driver (24) which can be actuated by an operator and arranged at said proximal handpiece (2), which driver (24) preferably is configured for being activated by a pressure applied in a longitudinal direction of the device.

16. The device (1; 100; 200) according to the preceding claim, wherein said proximal driver (24) comprises a button, preferably configured for being activated by the palm of the hand.

17. The device (1; 100; 200) according to anyone of the preceding claims, further comprising a suction system (5), configured in such a way as to apply a sucking action on the vaginal vault or other organ at a distal portion of said main body (3), in particular at a preferably lateral opening (32) thereof.

18. The device (1; 100; 200) according to anyone of the preceding claims, wherein said actuating means (10), said means for applying a staple (4) and/or said suction means (5) comprise an operative channel (37, 41, 51) extending longitudinally inside said main body (3).

19. The device (1) according to anyone of the preceding claims, comprising a connection (13) for a power supply and/or pneumatic suction system, preferably arranged at said proximal handpiece (2).

20. A surgical system comprising a device (1; 100; 200) according to anyone of the preceding claims and a supporting element, in particular a *mesh* (M), inserted inside said main body (3).

## Patentansprüche

1. Chirurgisches Gerät (1; 100; 200), das konfiguriert ist, um ein Stützelement (M), vorzugsweise ein Netz (*"mesh"*), an dem Scheidengewölbe, an dem Rektum oder an irgendeinem anderen Beckenorgan einzuführen, um dessen Prolaps zu behandeln, wobei das Gerät umfasst:
einen proximalen Griff (2), der von einem Operateur gehalten werden kann;
einen länglichen Hauptkörper (3), der mit dem Griff (2) verbunden oder fest verbindbar ist und diesbezüglich distal angeordnet ist, wobei der Hauptkörper (3) geeignet ist, perkutan durch den Unterleib hoch zu dem Scheidengewölbe oder dem anderen Organ eingeführt zu werden;
Kanalisiermittel (10), die an einem distalen Endbereich (31) des Hauptkörpers (3) angeordnet sind und an dem proximalen Griff (2) betreibbar sind, wobei die Kanalisiermittel (3) geeignet sind, alternativ eine minimale Belastungskonfiguration und eine maximale Belastungskonfiguration anzunehmen, geeignet zum Begünstigen der Trennung von Unterleibgeweben während des Einführens des Hauptkörpers (3) durch den Unterleib, **dadurch gekennzeichnet, dass** der Hauptkörper (3) einen länglichen Raum (30) aufweist, der geeignet ist, das Stützelement (M) zu beherbergen.

2. Gerät (1; 100; 200) nach Anspruch 1, wobei der Hauptkörper (3) eine Wellenform aufweist, die sich länglich gemäß einem Profil erstreckt, das geeignet ist, eine anatomische Kurve wiederzugeben, die zwischen der seitlichen Wand des Unterleibs und dem Scheidengewölbe vorhanden ist.

3. Gerät (1; 100; 200) nach einem der vorhergehenden Ansprüche, wobei der Hauptkörper (3) ein distales Ende mit einer Spitzenform (31) aufweist, insbesondere dreilappig, geeignet zum Begünstigen des Einführens des Geräts durch die Unterleibgewebe.

4. Gerät (1) nach einem der vorhergehenden Ansprüche, wobei die Konfigurationen einer maximalen und einer minimalen Belastung der Kanalisiermittel (10) einer maximalen Erweiterung beziehungsweise einer minimalen Erweiterung davon entsprechen, in einer Querrichtung senkrecht zu einer longitudinalen Richtung einer Vorwärtsbewegung des Hauptkörpers (3) durch den Unterleib.

5. Gerät (1) nach einem der vorhergehenden Ansprüche, wobei die Kanalisiermittel (10) ein oder mehrere Elemente (11) umfassen, die selektiv deformierbar sind, um die Konfigurationen einer maximalen und einer minimalen Belastung anzunehmen.

6. Gerät (1) nach einem der vorhergehenden Ansprüche, wobei die Kanalisiermittel (10) eine Membran oder einen Ballon (11) umfassen, welcher selektiv aufblasbar ist.

7. Gerät (100; 200) nach einem der Ansprüche 1 bis 5, wobei die Kanalisiermittel (110) eine Vielzahl reversibel deformierbarer Elemente (111) umfassen, die vorzugsweise konfiguriert sind, um selektiv jedes aus einer jeweiligen Öffnung (133) herauszuführen, die in einer Seitenschürze des Hauptkörpers (103) vorgesehen ist, um die maximale Belastungskonfiguration anzunehmen.

8. Gerät (100; 200) nach dem vorhergehenden Anspruch, wobei die deformierbaren Elemente eine Vielzahl longitudinaler Balkenelemente (111) umfassen, vorzugsweise aus Kunststoffmaterial gefertigt, oder eine Vielzahl longitudinaler fadenförmiger Elemente (211), vorzugsweise aus Metall gefertigt.

9. Gerät (200) nach Anspruch 7 oder 8, wobei die deformierbaren Elemente eine Gitterkonfiguration aufweisen, die durch longitudinale Elemente (211) und transversale Elemente (217) gebildet ist.

10. Gerät (100; 200) nach einem der Ansprüche 7 bis 9, das eine Welle (112) zum Betätigen der reversibel deformierbaren Elemente (111) umfasst, wobei die Betätigungswelle (112) sich longitudinal innerhalb des Hauptkörpers (103) erstreckt, wobei vorzugsweise die reversibel deformierbaren Elemente (111) distal mit Bezug auf die Betätigungswelle (112) angeordnet und daran gesichert sind.

11. Gerät (1; 100; 200) nach einem der vorhergehenden Ansprüche, welches Mittel (21; 121) zum Betätigen der Kanalisiermittel (10) umfasst, wobei die Betätigungsmittel (21; 121) an dem proximalen Griff (2) angeordnet sind, von einem Operateur aktiviert werden können und vorzugsweise einen Hebel oder eine Taste umfassen, wobei die Betätigungsmittel (21; 121) vorzugsweise konfiguriert sind, um durch eine Kraft aktiviert zu werden, die in einer Richtung transversal oder longitudinal mit Bezug auf das Gerät angewendet wird.

12. Gerät (1; 100; 200) nach dem vorhergehenden Anspruch, wobei die Betägigungsmittel (21) ein Betätigungselement (22) und ein elastisches Gegenmittel (23) umfassen, das damit assoziiert ist, wobei das elastische Gegenmittel (23) geeignet ist, das Betätigungsmittel (22) in eine Ruhekonfiguration zurückzubringen, die der minimalen Belastungskonfiguration der Kanalisiermittel (10) entspricht.

13. Gerät (1; 100; 200) nach einem der vorhergehenden Ansprüche, das Mittel (4) zum Zufügen einer Heftung oder eines Befestigungspunktes des Stützelements an dem Scheidengewölbe oder dem anderen Organ umfasst.

14. Gerät (1; 100; 200) nach dem vorhergehenden Anspruch, wobei die Mittel zum Zufügen einer Heftung einen Führungskanal (41) für die Heftung umfassen, der sich in dem Hauptkörper (3) erstreckt und vorzugsweise mit einer Ausgangsöffnung (42) des Punktes ausgestattet ist, der an einer distalen Öffnung (32) des Hauptkörpers (3) angeordnet ist, vorzugsweise einer Seitenöffnung.

15. Gerät (1; 100; 200) nach Anspruch 13 oder 14, wobei die Mittel (4) zum Zufügen einer Heftung eine Antriebseinrichtung (24) umfassen, die von einem Operateur betätigt werden kann und an dem proximalen Griff (2) angeordnet ist, wobei die Antriebseinrichtung (24) vorzugsweise konfiguriert ist, um durch einen Druck aktiviert zu werden, der in einer longitudinalen Richtung des Geräts aufgebracht wird.

16. Gerät (1; 100; 200) nach dem vorhergehenden Anspruch, wobei die proximale Antriebseinrichtung (24) eine Taste umfasst, die vorzugsweise konfiguriert ist, um durch die Fläche der Hand aktiviert zu werden.

17. Gerät (1; 100; 200) nach einem der vorhergehenden Ansprüche, das weiterhin ein Saugsystem (5) umfasst, das auf eine solche Weise konfiguriert ist, dass es eine Saugwirkung auf das Scheidengewölbe oder das andere Organ an einem distalen Bereich des Hauptkörpers (3) aufbringt, insbesondere an einer vorzugsweise seitlichen Öffnung (32) davon.

18. Gerät (1; 100; 200) nach einem der vorhergehenden Ansprüche, wobei die Betätigungsmittel (10), die Mittel zum Zuführen einer Heftung (4) und/oder die Saugmittel (5) einen Betriebskanal (37, 41, 51) umfassen, der sich longitudinal innerhalb des Hauptkörpers (3) erstreckt.

19. Gerät (1) nach einem der vorhergehenden Ansprüche, das eine Verbindung (13) für eine Energieversorgung und/oder ein pneumatisches Saugsystem umfasst, vorzugsweise angeordnet an dem proximalen Griff (2).

20. Chirurgisches System mit einem Gerät (1; 100; 200) nach einem der vorhergehenden Ansprüche und einem Stützelement, vorzugsweise einem Netz (*"mesh"*) (M), das in den Hauptkörper (3) eingeführt ist.

## Revendications

1. Dispositif chirurgical (1 ; 100; 200) configuré pour insérer un élément de support (M), de préférence une maille, au niveau du dôme vaginal, du rectum et de n'importe quel autre organe pelvien afin de traiter son prolapsus, lequel dispositif comprend :
une pièce à main proximale (2) apte à être maintenue par un opérateur ;
un corps principal allongé (3), raccordé ou pouvant être raccordé de manière fixe à ladite pièce à main (2) et agencé de manière distale par rapport à ce dernier, lequel corps principal (3) est approprié pour être inséré par voie percutanée à travers l'abdomen jusqu'au dôme vaginal ou l'autre organe ;
un moyen de canalisation (10) agencé au niveau de la partie d'extrémité distale (31) dudit corps principal (3) et pouvant fonctionner au niveau de ladite pièce à main proximale (2), lequel moyen de canalisation (3) est apte à adopter, de manière alternée, une configuration d'encombrement minimum et une configuration d'encombrement maximum apte à favoriser la séparation des tissus abdominaux pendant l'insertion dudit corps principal (3) à travers l'abdomen, **caractérisé en ce que** le corps principal (3) à un espace longitudinal (30) apte à loger l'élément de support (M).

2. Dispositif (1 ; 100; 200) selon la revendication 1, dans lequel ledit corps principal (3) a une forme ondulée, s'étendant de manière longitudinale selon un profil apte à reproduire une courbe anatomique comprise entre la paroi latérale de l'abdomen et le dôme vaginal.

3. Dispositif (1; 100; 200) selon l'une quelconque des revendications précédentes, dans lequel ledit corps principal (3) a une extrémité distale avec une forme pointue (31), en particulier à trois lobes, apte à favoriser l'insertion du dispositif à travers les tissus abdominaux.

4. Dispositif (1) selon l'une quelconque des revendications précédentes, dans lequel lesdites configurations d'encombrement maximum et minimum dudit moyen de canalisation (10) correspondent à son extension maximum et à son extension minimum, respectivement, dans une direction transversale orthogonale à une direction longitudinale de mouvement vers l'avant dudit corps principal (3) à travers l'abdomen.

5. Dispositif (1) selon l'une quelconque des revendications précédentes, dans lequel ledit moyen de canalisation (10) comprend un ou plusieurs éléments (11) sélectivement déformables pour adopter lesdites configurations d'encombrement maximum et minimum.

6. Dispositif (1) selon l'une quelconque des revendications précédentes, dans lequel ledit moyen de canalisation (10) comprend une membrane ou un ballonnet (11) qui est sélectivement gonflable.

7. Dispositif (100 ; 200) selon l'une quelconque des revendications 1 à 5, dans lequel ledit moyen de canalisation (110) comprend une pluralité d'éléments réversiblement déformables (111), de préférence configurés pour sortir sélectivement chacun par une extrémité (133) respective obtenue dans une jupe latérale dudit corps principal (103) pour adopter ladite configuration d'encombrement maximum.

8. Dispositif (100; 200) selon la revendication précédente, dans lequel lesdits éléments déformables comprennent une pluralité d'éléments de barre longitudinaux (111), de préférence réalisés avec un matériau plastique, ou une pluralité d'éléments filiformes longitudinaux (211) de préférence réalisés à partir de métal.

9. Dispositif (200) selon la revendication 7 ou 8, dans lequel lesdits éléments déformables ont une configuration de grille formée par des éléments longitudinaux (211) et des éléments transversaux (217).

10. Dispositif (100 ; 200) selon l'une quelconque des revendications 7 à 9, comprenant un arbre (112) pour actionner lesdits éléments réversiblement déformables (111), lequel arbre d'actionnement (112) s'étend longitudinalement à l'intérieur dudit corps principal (103), dans lequel de préférence lesdits éléments réversiblement déformables (111) sont agencés de manière distale par rapport audit arbre d'actionnement (112) et fixés à ce dernier.

11. Dispositif (1; 100; 200) selon l'une quelconque des revendications précédentes, comprenant des moyens (21; 121) pour actionner ledit moyen de canalisation (10), lequel moyen d'actionnement (21 ; 121) est agencé au niveau de ladite pièce à main proximale (2), il peut être activé par un opérateur et de préférence il comprend un levier ou un bouton, lequel moyen d'actionnement (21 ; 121) est de préférence configuré pour être activé par une force appliquée dans une direction transversale ou longitudinale par rapport au dispositif.

12. Dispositif (1 ; 100 ; 200) selon la revendication précédente, dans lequel ledit moyen d'actionnement (21) comprend un élément d'actionnement (22) et un moyen élastique de contraste (23) associé à ce dernier, lequel moyen élastique de contraste (23) est apte à ramener ledit élément d'actionnement (22) dans une configuration de repos correspondant à ladite configuration d'encombrement minimum dudit moyen de canalisation (10).

13. Dispositif (1; 100; 200) selon l'une quelconque des revendications précédentes, comprenant un moyen (4) pour appliquer une agrafe ou un point de fixation de l'élément de support sur le dôme vaginal ou un autre organe.

14. Dispositif (1 ; 100 ; 200) selon la revendication précédente, dans lequel ledit moyen (4) pour appliquer une agrafe comprend un canal de guidage (41) pour l'agrafe s'étendant à l'intérieur dudit corps principal (3) et de préférence prévu avec un orifice de sortie (42) du point agencé au niveau d'une ouverture distale (32), de préférence une ouverture latérale, dudit corps principal (3).

15. Dispositif (1 ; 100 ; 200) selon la revendication 13 ou 14, dans lequel ledit moyen (4) pour appliquer une agrafe comprend un dispositif d'entraînement (24) qui peut être actionné par un opérateur et agencé au niveau de ladite pièce à main proximale (2), lequel dispositif d'entraînement (24) est de préférence configuré pour être activé par une pression appliquée dans une direction longitudinale du dispositif.

16. Dispositif (1 ; 100 ; 200) selon la revendication précédente, dans lequel ledit dispositif d'entraînement proximal (24) comprend un bouton, de préférence configuré pour être activé par la paume de la main.

17. Dispositif (1; 100; 200) selon l'une quelconque des revendications précédentes, comprenant en outre un système d'aspiration (5), configuré afin d'appliquer une action d'aspiration sur le dôme vaginal ou un autre organe au niveau d'une partie distale dudit corps principal (3), en particulier au niveau de son extrémité de préférence latérale (32).

18. Dispositif (1; 100; 200) selon l'une quelconque des revendications précédentes, dans lequel ledit moyen d'actionnement (10), ledit moyen pour appliquer une agrafe (4) et/ou ledit moyen d'aspiration (5) comprennent un canal opérationnel (37, 41, 51) s'étendant de manière longitudinale à l'intérieur dudit corps principal (3).

19. Dispositif (1) selon l'une quelconque des revendications précédentes, comprenant un raccordement (13) pour un système d'alimentation électrique et/ou d'aspiration pneumatique, de préférence agencé au niveau de ladite pièce à main proximale (2).

20. Système chirurgical comprenant un dispositif (1 ; 100; 200) selon l'une quelconque des revendications précédentes, et un élément de support, en particulier une maille (M) insérée à l'intérieur dudit corps principal (3).
